# EUROPEAN PATENT APPLICATION

(11) **EP 2 255 792 A1**
(43) Date of publication of application: **01.12.2010**
(21) Application number: 09006789.3
(22) Date of filing: 20.05.2009
(51) Int. Cl.: A61K 9/20, A61K 9/48

(54) **Pharmaceutical compositions for N-[2-(Diethylamino)ethyl]5-[(fluoro-1,2-dihydro-2-oxo-3H-indole-3-ylidene) methyl]-2,4-dimenthyl-1H-pyrrole-3-carboxamide**

(71) Applicant: Ratiopharm GmbH, 89079 Ulm (DE)
(72) Inventor: Stefan, Ralph, D-88370 Ebenweiler (DE); Holfinger, Konstantin, 88326 Aulendorf (DE); Heinze, Liane, D-61440 Oberursel (DE)
(74) Representative: Teipel, Stephan

(57) **Abstract**

The present invention refers to pharmaceutical compositions comprising N-[2-(diethylamino)ethyl]-5-[(fluoro-1,2-dihydro-2-oxo-3H-indole-3-ylidene)methyl]-2,4-dimethyl-1 H-pyrrole-3-carboxamide as active pharmaceutical ingredient.

## Description

### Abstract

The present invention refers to pharmaceutical compositions comprising N-[2-(diethylamino)ethyl]-5-[(fluoro-1,2-dihydro-2-oxo-3H-indole-3-ylidene)methyl]-2,4-dimethyl-1H-pyrrole-3-carboxamide as active pharmaceutical ingredient.

### Background of the invention

The compound N-[2-(diethylamino)ethyl]-5-[(5-fluoro-1,2-dihydro-2-oxo-3H-indol-3-ylidene)methyl]-2,4-dimethyl-1H-pyrrole-3-carboxamide (herein also referred to as Sunitinib) is a receptor tyrosine kinase inhibitor which is used to treat disorders like renal cell carcinoma (RCC) and gastrointestinal stromal tumors (GIST). Its activity relies on the inhibition of cellular signaling by targeting multiple receptor tyrosine kinases including platelet-derived growth factor receptors and vascular endothelial growth factor receptors. Since both kinds of receptors are involved in tumor angiogenesis and tumor cell proliferation, the simultaneous inhibition of these targets results in both reduced tumor vascularization and cancer cell death. These effects are responsible for the finally observed shrinkage of the renal cell carcinoma and gastrointestinal stromal tumors, respectively.

WO 01/60814 discloses two processes of preparing Sunitinib. According to these and other known manufacturing processes, Sunitinib is obtained as a solid. One of the forms of Sunitinib is its crystalline malic acid salt as described in WO 03/016305.

Sunitinib and its pharmaceutical effects on disorders like cancer are described in WO 01/60814. Further medical uses of Sunitinib and its salts are inter alia known from WO 01/45689 and WO 03/035009.

Typically, Sunitinib is administered in a dose of 50 mg once daily, which, if necessary, has to be varied according to individual tolerance and safety. Thus, in order to obtain sufficiently flexible dosing, individual dosage forms contain 12.5, 25, 37.5 and 50 mg Sunitinib. Capsules comprising the malate salt of Sunitinib are sold under the brand name Sutent^{®} (by Pfizer Pharma).

These capsules are unnecessarily big, and capsules in general are hard to swallow, especially for cancer patients whose medications usually consist of multiple drug regimen demanding the administration of large numbers of tablets or capsules often along with an intravenous therapy. Further, these patients often suffer from nausea and lesions of the oral mucosa. Therefore the peroral application of drugs may be hampered by vomiting fits and swallowing problems. Hence, it would be desirable to reduce the size of capsules or tablets thereby facilitating the administration of the cancer patients' daily medication.

Still, good homogeneity, stability, compressibility, requisite flow and cohesive characteristics, dissolution and cost efficiency have to be warranted.

It has been found that the above and other requirements can be dealt with by providing a pharmaceutical composition in the form of micro tablets optionally contained in a capsule shell. A plurality of micro tablets can be easily handled and filled into capsule shells. Moreover, the micro tablets are comparably small and, thus, allow for the use of small capsules. A further advantage is that small micro tablets can be provided with a coating to tailor the dissolution profile of the composition. Micro tablets with the same or different coatings can be combined within one capsule. The variability of the composition is further increased by the possibility to combine micro tablets having different contents of Sunitinib within one capsule. Moreover, it has been found that the dissolution of the pharmaceutical composition of the present invention is faster than the dissolution of commercial Sunitinib tablets.

Thus, the present invention relates to pharmaceutical compositions comprising N-[2-(di-ethylamino)ethyl]-5-[(5-fluoro-1,2-dihydro-2-oxo-3H-indol-3-ylidene)methyl]-2,4-dimethyl-1H-pyrrole-3-carboxamide or a hydrate, solvate, polymorph or salt thereof as active pharmaceutical ingredient, wherein the active pharmaceutical ingredient is contained in micro tablets, which are optionally contained in a capsule shell.

### Abbreviations

- A: Ångström
- DSC: differential scanning calorimetry
- DMSG: dynamic moisture sorption gravimetry
- IR: infrared
- RH: relative humidity
- RT: room temperature
- SCXRD: singe crystal X-ray diffraction
- XRPD: X-ray powder diffraction

### Brief description of the figures

Figure 1: XRPD pattern of Sunitinib in the polymorphic form I
Figure 2: XRPD pattern of Sunitinib in the polymorphic form II
Figure 3: XRPD pattern of Sunitinib in the polymorphic form III
Figure 4: DSC thermogram of Sunitinib in the polymorphic form I

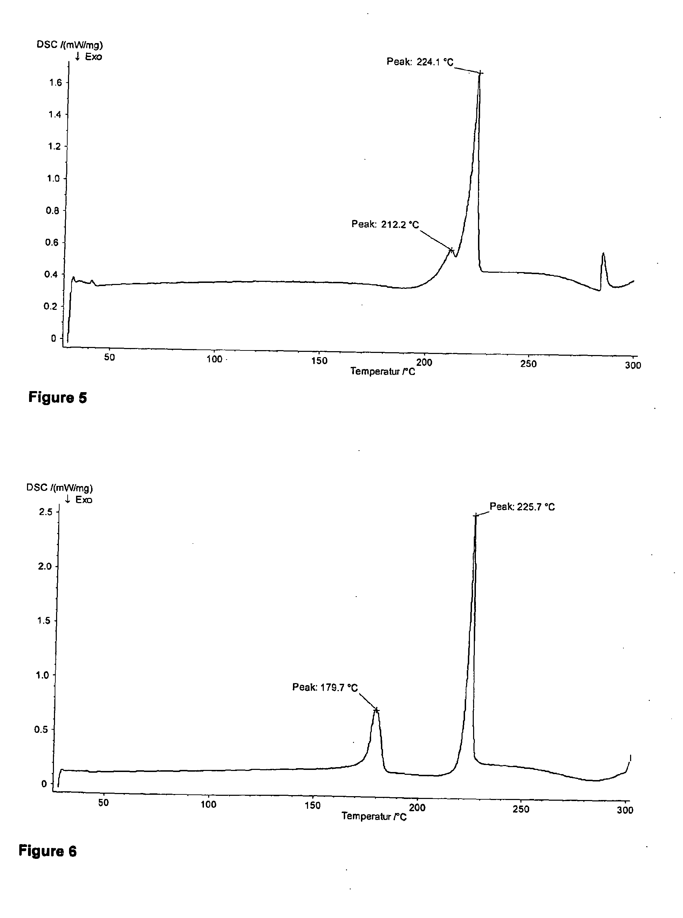

Figure 5: DSC thermogram of Sunitinib in the polymorphic form II
Figure 6: DSC thermogram of Sunitinib in the polymorphic form III

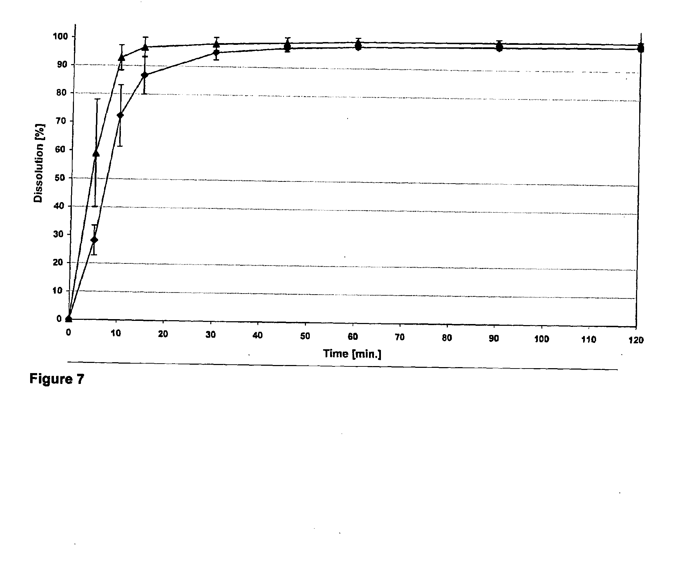

Figure 7: Dissolution profile of the formulation described in the example (triangles) compared to Sutent^{®} capsules (rhombi) (conditions: 900 ml phosphate buffer pH 3.2, 37°C, 50 rpm paddle, spiral capsule sinker)

### Description of the invention

N-[2-(Diethylamino)ethyl]-5-[(5-fluoro-1,2-dihydro-2-oxo-3H-indol-3-ylidene)methyl]-2,4-dimethyl-1 H-pyrrole-3-carboxamide (Sunitinib) has the following chemical structure:

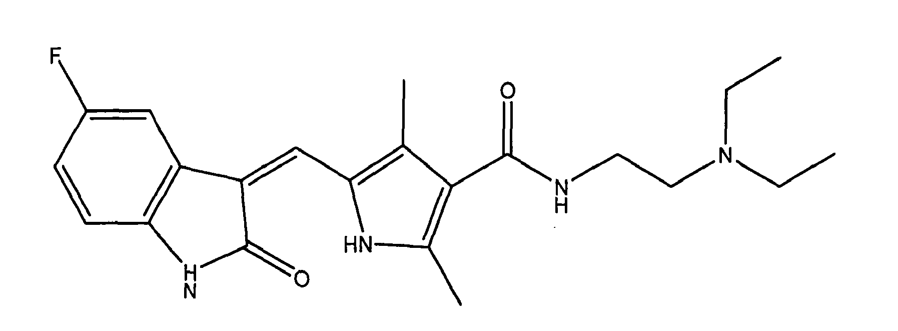

Sunitinib can be readily synthesized using techniques well known in the art. Syntheses of Sunitinib are disclosed for example in WO 01/60814. Sunitinib can be obtained in a yellow to greenish yellow powder exhibiting a very low hygroscopicity. In the following, this crystal form of Sunitinib will be designated as "polymorphic form I" or "polymorph I". This powder is fine particulate, hard to collect by filtration, highly electrostatic and tends to strongly stick to all kinds of surfaces.

The malic acid salt as well as methods for preparing it are described in WO 03/016305.

Polymorphic forms of Sunitinib in its free base form as well as processes for their manufacture are disclosed in European patent application 080041443.7.

The term "active pharmaceutical ingredient " (API) refers to Sunitinib, in particular crystalline Sunitinib and to salts, polymorphs, solvates or hydrates thereof.

The term "crystalline" refers to any substantially, preferably completely non amorphous forms of the active pharmaceutical ingredient. The term "amorphous form" refers to a form of active pharmaceutical ingredient which has no long-range order like crystalline forms. The atoms or molecules of a material present in amorphous form are arranged in a non-uniform array. It is for example possible to distinguish amorphous from crystalline forms of a compound by powder X-ray diffraction.

Preferably, at least 60 wt.%, at least 70 wt.% or at least 80 wt.% of the active pharmaceutical ingredient are present in crystalline form, even more preferably at least 90 wt.%, most preferably at least 95 wt.%, wherein the respective amounts are being referred to the total weight of the active pharmaceutical ingredient in the composition of the present invention.

The term "pharmaceutical composition" refers to single dosage forms, such as tablets, capsules, sachets, etc., as well as micro tablets which are used in the preparation of single dosage forms. Where it is referred to the total weight of the pharmaceutical composition and the pharmaceutical composition in a single dosage form the total weight is the weight of the single dosage form excluding, if applicable, the weight of any coating or capsule shell.

The term "micro tablet" refers to a small tablet obtained according to the standard compression methods known in the art. The weight of a micro tablet is typically in the range of 15 to 200 mg, preferably of 20 to 150 mg, more preferably 20 to 100 mg.

The active pharmaceutical ingredient may be provided in form of any pharmaceutically acceptable salt, preferably as the malate salt, in the form of the free base or solvates, hydrates or polymorphs thereof, preferably in its polymorphic form I, II or III, more preferably in its polymorphic form II or III, most preferably in its polymorphic form III.

### Polymorph I of Sunitinib

Polymorph I of Sunitinib is characterized by an XRPD pattern having a characteristic peak at 4.5 ± 0.2 degrees 2-theta, in particular with peaks at 4.5 ± 0.2; 9.1 ± 0.2; 16.8 ± 0.2 and 26.0 ± 0.2 degrees 2-theta.

Furthermore, polymorph I of Sunitinib can be characterized by an XRPD pattern with peaks at 4.5 ± 0.2; 9.1 ± 0.2; 15.2 ± 0.2; 16.8 ± 0.2; 18.3 ± 0.2; 20.4 ± 0.2; 21.8 ± 0.2; 22.9 ± 0.2 and 26.0 ± 0.2 degrees 2-theta.

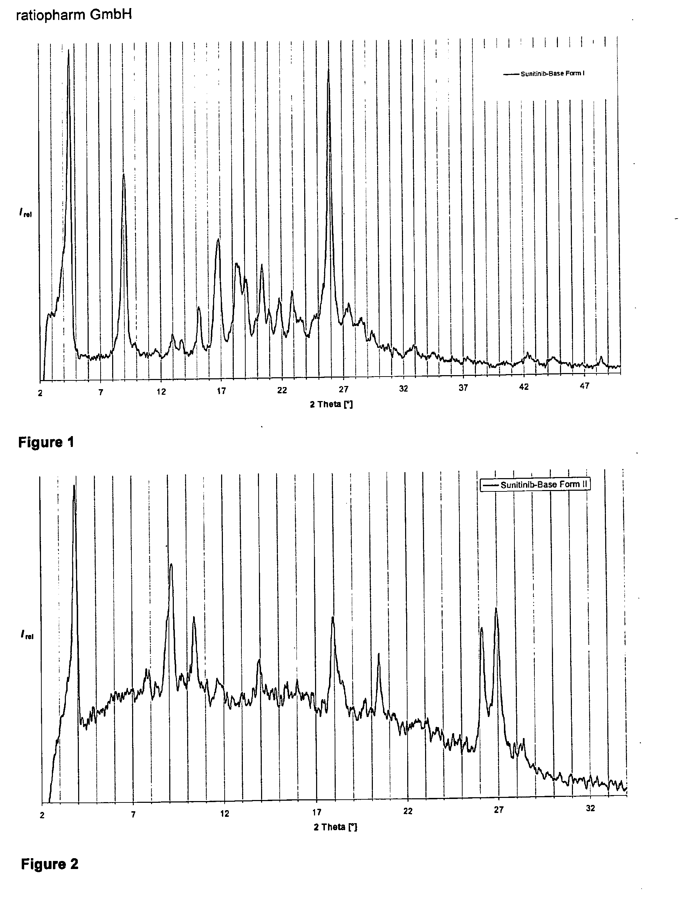

The XRPD pattern of polymorph I of Sunitinib is shown in figure 1.

Polymorph I of Sunitinib shows a Raman spectrum exhibiting characteristic peaks at 2927 ± 2 cm⁻¹, 1679 ± 2 cm⁻¹, 1585 ± 2 cm⁻¹, 1437 ± 2 cm⁻¹, 1334 ± 2 cm⁻¹, 1278 ± 2 cm⁻¹ and 670 ± 2cm⁻¹_{.}

Polymorph I of Sunitinib shows an IR spectrum exhibiting characteristic peaks at 2969 ± 2 cm⁻¹ 1479 ± 2 cm⁻¹, 1330 ± 2 cm⁻¹, 1189 ± 2 cm⁻¹, 797 ± 2 cm-¹, 667 ± 2 cm⁻¹ and 609 ± 2 cm⁻¹.

Polymorph I of Sunitinib exhibits a low hygroscopicity. DMSG revealed a moisture sorption of maximum about 1 % referred to the weight of the sample.

By crystallization and/or recrystallization of Sunitinib from different solvents and mixtures of two or more solvents further polymorphic forms are obtained. While polymorph I of Sunitinib exhibits low hygroscopicity it has unfavourable properties such as an extremely fine particularity and bad filterability, stickiness to all sorts of surfaces and electrostatic properties hampering the industrial workability including the development of galenical formulations. In the handling of the further polymorphs of Sunitinib the above mentioned problems in filterability, electrostatic properties and stickiness are not encountered.

### Polymorph II of Sunitinib

Polymorph II of Sunitinib has the following characteristics:

| | |
|---|---|
| • crystal system | triclinic |
| • space group | P-1 |
| • cell metrics | a = 13.5 ± 0.2 Å |
| | b = 14.4 ± 0.2 Å |
| | c = 24.3 ± 0.2 Å |
| | α = 79 ± 1° |
| | β = 81 ± 1° |
| | Y = 89 ± 1° |
| • cell volume V | 4598.85 Ǻ³ |
| • molecules per unit cell | 8 |

Polymorph II of Sunitinib is characterized by an XRPD pattern having a characteristic peak at 3.8 ± 0.2 degrees of 2-theta, in particular with peaks at 3.8 ± 0.2; 9.0 ± 0.2; 14.0 ± 0.2; 18.1 ± 0.2 and 20.5 ± 0.2 degrees 2-theta.

Furthermore, polymorph II of Sunitinib can be characterized by an XRPD pattern with peaks at 3.8 ± 0.2; 9.0 ± 0.2; 14.0 ± 0.2; 18.1 ± 0.2; 20.5 ± 0.2; 26.6 ± 0.5 and 27.5 ± 0.6 degrees 2-theta.

The XRPD of polymorph II of Sunitinib is shown in figure 2.

Polymorph II of Sunitinib shows a Raman spectrum exhibiting characteristic peaks at 2929 ± 2 cm⁻¹, 1627 ± 2 cm⁻¹, 1583 ± 2 cm⁻¹, 1425 ± 2 cm⁻¹, 1328 ± 2 cm⁻¹, 1285 ± 2 cm⁻¹, 1264 ± 2 cm⁻¹ and 669 ± 2 cm⁻¹.

Polymorph II of Sunitinib shows an IR spectrum exhibiting characteristic peaks at 1667 ± 2 cm⁻¹, 1476 ± 2 cm⁻¹, 1325 ± 2 cm⁻¹, 1147 ± 2 cm⁻¹, 794 ± 2 cm⁻¹, 668 ± 2 cm⁻¹ and 608 ± 2 cm⁻¹.

Sunitinib in the polymorphic form II exhibits some hygroscopicity. DMSG revealed a moisture sorption of more than 6 % referred to the weight of the sample.

Polymorph II of Sunitinib can eb prepared by a process comprising the steps of:
- dissolving Sunitinib present in any polymorphic or amorphous form or mixtures thereof, preferably in its polymorphic form I in a suitable inert solvent, preferably in water or an organic solvent or a mixture of two or more suitable inert solvents, preferably at a temperature between 25 °C and the reflux temperature of the solvent or the solvent mixture,
- optionally adding another solvent, preferably another organic solvent,
- concentrating the solution, preferably under reduced pressure, more preferably at a temperature between 25 °C and the reflux temperature of the solvent or the solvent mixture, most preferably under reduced pressure at 25 to 60 °C,
- collecting the resulting crystalline precipitate, preferably by filtration.

The crystallization is carried out in a suitable inert solvent or in a mixture of two or more suitable inert solvents. Examples of such suitable inert solvents are water, aliphatic and aromatic hydrocarbons (preferably hexane, benzene, toluene or xylene), aliphatic alcohols (preferably methanol, ethanol, propanol, iso-propanol), ethers (preferably diethyl ether, diisopropyl ether or dimethoxyethane), cyclic ethers (preferably tetrahydrofuran or dioxane), ketones (preferably acetone, methylisobutylketone or methylethylketone), esters (preferably ethylacetate), chlorinated hydrocharbons (preferably dichloromethane or chloroform) or nitrogen containing organic solvents (preferably N-methyl pyrollidone, dimethylformamide or acetonitrile). Acetone and toluene are especially preferred.

Polymorph II of Sunitinib can be prepared by an alternative process comprising the steps of:
- dissolving a salt of Sunitinib, preferably a salt of Sunitinib (for example as disclosed in EP 1255752 B1), more preferably the malate salt, in a suitable inert solvent, preferably in water, or a mixture of two or more suitable inert solvents optionally heating the solvent to a temperature between 25 °C and the reflux temperature of the solvent or the solvent mixture,
- adding a base, preferably NaOH,
- collecting the resulting crystalline precipitate, preferably by filtration.

As solvents the suitable inert solvents as described above can be used.

The above described reaction is carried out in the presence of a base, preferably a Bronsted base. Examples of suitable Bronsted bases are metal hydroxides, metal carbonates or amines, preferably alkali metal hydroxides, alkali metal carbonates, alkali metal bicarbonates, ammonia, or organic amines, such as, for example, sodium hydroxide, potassium hydroxide, sodium carbonate, potassium carbonate, sodium bicarbonate, ammonia, diethylamine, triethylamine, diisopropylamine or pyridine. Sodium hydroxide is preferred. The base is used in an amount sufficient to obtain the free base of Sunitinib from its salt.

### Polymorph III of Sunitinib

Polymorph III of Sunitinib has the following characteristics:

| | |
|---|---|
| • crystal system | monoclinic |
| • space group | P 2₁/n |
| • cell metrics | a = 4.97560(10) Å |
| | b = 28.1365(6) Å |
| | c = 14.5880(3) Å |
| | β = 93.5130(10)° |
| • cell volume V | 2038.42(7) Ǻ³ |
| • molecules per unit cell | 4 |

Polymorph III of Sunitinib is characterized by an XRPD pattern having a characteristic peak at 6.3 ± 0.2 degrees 2-theta, in particular with peaks at 6.3 ± 0.2; 22.2 ± 0.2 and 26.4 ± 0.2 degrees 2-theta.

Furthermore, polymorph III of Sunitinib can be characterized by an XRPD pattern with peaks at 6.3 ± 0.2; 14.0 ± 0.2; 15.4 ± 0.2, 18.9 ± 0.2, 19.3 ± 0.2; 22.2 ± 0.2; 24.2 ± 0.2 and 26.4 ± 0.2 degrees 2-theta.

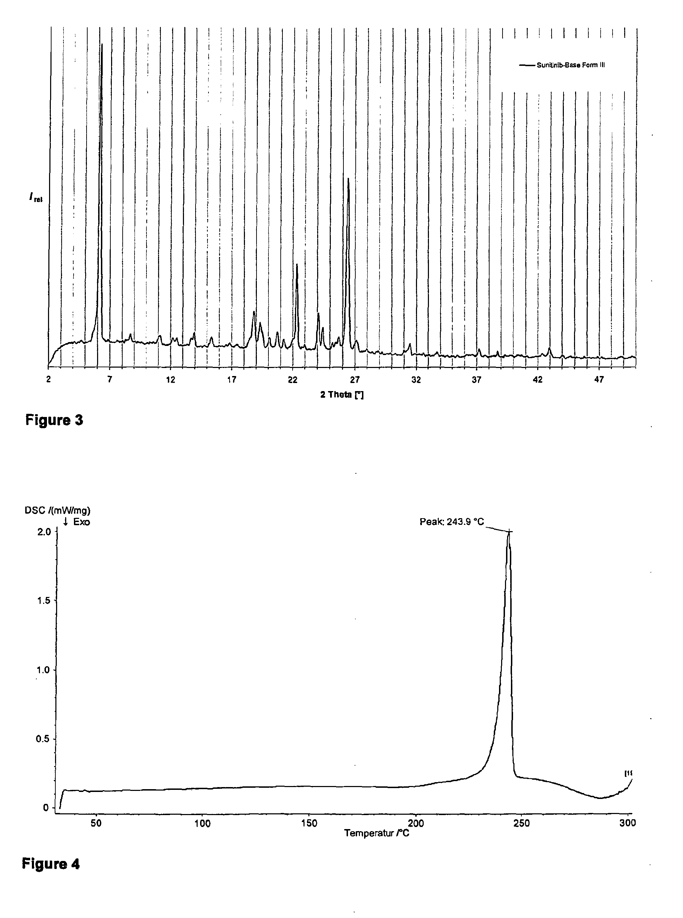

The XRPD pattern of polymorph III of Sunitinib is shown in figure 3.

Polymorph III of Sunitinib shows a Raman spectrum exhibiting characteristic peaks at 1674 ± 2 cm⁻¹, 1569 ± 2 cm⁻¹, 1414 ± 2 cm⁻¹, 1327 ± 2 cm⁻¹, 1297 ± 2 cm⁻¹, 1259 ± 2 cm⁻¹, 1030 ± 2 cm⁻¹ and 666 ± 2 cm⁻¹_{.}

Polymorph III of Sunitinib shows an IR spectrum exhibiting characteristic peaks at 3435 ± 2 cm⁻¹, 1670 ± 2 cm⁻¹, 1473 ± 2 cm⁻¹, 1294 ± 2 cm⁻¹, 1194 ± 2 cm⁻¹, 1146 ± 2 cm⁻¹, 786 ± 2 cm¹, 663 ± 2 cm⁻¹, 617 ± 2 cm⁻¹.

Polymorph III exhibits a very low hygroscopicity. Under DMSG, the sample does not gain weight, even at a relative humidity of 95 %.

Polymorph III is preferably further characterized by the absence of any solvent molecules within the crystal.

Polymorph III of Sunitinib can be prepared by a process comprising the steps of:
- preparing a clear saturated solution of Sunitinib in a suitable inert solvent,
- cooling the solution and allowing Sunitinib to crystallize,
- removing the resulting precipitate,
- keeping the filtrate at room temperature until Sunitinib crystallizes in the form of dark
   orange needles
   or alternatively:
- preparing a clear saturated solution of Sunitinib in a suitable inert solvent,
- cooling the solution and seeding it with crystals of polymorph III of Sunitinib,
- allowing polymorph III to crystallize from the seeded solution at room temperature in the form of dark orange needles.

Preferably the process comprises the steps of:
- preparing a suspension of Sunitinib by suspending Sunitinib present in any polymorphic or amorphous form or mixtures thereof, preferably in its polymorphic form I or II, in a suitable inert solvent, preferably in an organic solvent or a mixture of two or more organic solvents,
- heating the suspension, preferably to the reflux temperature of the solvent or the solvent mixture, optionally by adding an additional amount of solvent, until a clear and saturated solution is obtained,
- cooling the solution and allowing Sunitinib to precipitate,
- removing the resulting precipitate, preferably by filtration,
- keeping the filtrate at room temperature until Sunitinib crystallizes in the form of dark
   orange needles,
   or alternatively:
- preparing a suspension of Sunitinib by suspending Sunitinib present in any polymorphic or amorphous form or mixtures thereof, preferably in its polymorphic form I or II in a suitable inert solvent, preferably in an organic solvent or a mixture of two or more organic solvents,
- heating the saturated suspension, preferably to the reflux temperature of the solvent or the solvent mixture, optionally by adding an additional amount of solvent, until a clear and saturated solution is obtained,
- cooling the solution and seeding it with crystals of polymorph III of Sunitinib,
- allowing polymorph III of Sunitinib to crystallize from the seeded solution at room temperature in the form of dark orange needles.

The above described crystallizations are carried out in suitable inert solvents or mixtures of two or more suitable inert solvents. Examples of such suitable inert solvents are water, aliphatic and aromatic hydrocarbons (preferably hexane, benzene, toluene or xylene), aliphatic alcohols (preferably methanol, ethanol, propanol, iso-propanol), ethers (preferably diethyl ether, diisopropyl ether or dimethoxyethane), cyclic ethers (preferably tetrahydrofuran or dioxane), ketones (preferably acetone, methylisobutylketone or methylethylketone), esters (preferably ethylacetate), chlorinated hydrocharbons (preferably dichloromethane or chloroform) or nitrogen containing organic solvents (preferably N-methyl pyrollidone, dimethylformamide or acetonitrile). Acetone and toluene are especially preferred. Advantageous properties as required are achieved if the active pharmaceutical ingredient is present in an amount of 10 to 90, preferably 20 to 80 %, more preferably 30 to 70 % most preferably 45 to 65 % by weight of the total weight of the composition.

The pharmaceutical composition of the present invention may further comprise one or more pharmaceutically acceptable excipients, such as fillers, binding agents, lubricants, flow enhancers, antisticking agents, disintegrating agents and solubilizers. As pharmaceutically acceptable excipients conventional excipients known to the person skilled in the art may be used. See for example "Lexikon der Hilfsstoffe für Pharmazie, Kosmetik und angrenzende . Gebiete", edited by H. P. Fiedler, 4th Edition, Edito Cantor, Aulendorf and earlier editions, and "Handbook of Pharmaceutical Excipients", Third Edition, edited by Arthur H. Kibbe, American Pharmaceutical Association, Washington, USA, and Pharmaceutical Press, London.

Preferred examples of the fillers are lactose, mannitol, sorbitol or microcrystalline cellulose. The filler is suitably present in an amount of 0 to 90 wt.%, preferably of 30 to 80 wt.% of the total weight of the composition.

The binding agent can for example be microcrystalline cellulose (MCC) and modified MCC, polyvinylpyrrolidone (PVP) or hydroxypropylmethyl cellulose (HPMC). Preferably the binding agent is present in an amount of 1 to 25 wt.%, more preferably at 2 to 10 wt.% of the total weight of the composition.

The lubricant is preferably a stearate, more preferably an earth alkali metal stearate, such as magnesium stearate. The lubricant is suitably present in an amount of 0.1 to 2 wt.%, preferably about 1 wt.% of the total weight of the composition.

Preferred disintegrating agents are croscarmellose sodium, sodium carboxymethyl starch or cross-linked polyvinylpyrrolidone (crospovidone). The disintegrating agent is suitably present in an amount of 0.1 to 20 wt.%, more preferably at about 0.5 to 7 wt.% of the total weight of the composition.

The flow enhancer can for example be colloidal silicon dioxide. Preferably, the flow enhancer is present in an amount of 0.5 to 8 wt.%, more preferably at 0.5 to 3 wt.% of the total weight of the composition.

The antisticking agent is for example talcum and may be present in amounts of 1 to 5 %.wt, more preferably in an amount of 1.5 to 3 wt.% of the total weight of the composition.

An improvement of the solubility of the active pharmaceutical ingredient can for example be achieved by the addition of complex forming agents/compounds (e.g. sodium benzoate, sodium salicylate or cyclodextrins), alternation of solvent properties (e.g. by adding PVP or polyethylene glycols) or the addition of solubilizers which form tenside micelles (e.g. surfactants).

Suitable solubilizers are for example surfactants such as polyoxyethylene alcohol ethers (e.g. Brij®), polysorbates (e.g. Tween®) or polyoxypropylene polyoxyethylene copolymers (poloxamer; e.g. Pluronic®) and may be present in amounts of 0.5 to 7 %.wt, more preferably 1 to 5 %.wt. of the total weight of the composition.

Alternatively, a pseudo-emulsifier can be used. Its mechanism of action mainly relies on an enhancement of viscosity. However, pseudo-emulsifiers also possess emulsifying properties. Preferred pseudo-emulsifiers of the present invention are for example cellulose ethers, gum Arabic or tragacanth and may be present in amounts of 1 to 10 %.wt, more preferably 3 to 7 %.wt. of the total weight of the composition.

A person skilled in the art may use these or other excipients in regard to the selected process of preparing the pharmaceutical compositions of the invention.

The micro tablets of the present invention can be formulated in any known matter, such as tablets, capsules or sachets. Preferably the micro tablets are contained in a capsule shell.

The pharmaceutical composition may contain a dosage amount of 12.5, 25, 37.5 or 50 mg of the active pharmaceutical ingredient referred to the weight of the free base of Sunitinib. Thus the administered amount can be readily varied according to individual tolerance and safety warranting more flexible dosing than the standard dose of 50 mg once daily.

The pharmaceutical composition of the present invention can be manufactured according to standard methods known in the art. For example, granules can be obtained by dry compaction or wet granulation. These granules can subsequently be mixed with e.g. suitable disintegrating agents, glidants and lubricants and compressed into micro tablets.

Micro tablets can also be obtained by direct compression of a suitable powder mixture, i.e. without any preceding granulation of the excipients.

Suitable powder or granule mixtures are further obtainable by spray drying, lyophilization, melt extrusion, pellet layering, coating of the active pharmaceutical ingredient or any other suitable. The so obtained powders or granulates can be mixed with one or more suitable ingredients and the resulting mixtures can be compressed to form micro tablets.

The above mentioned methods known in the art also include grinding and sieving techniques permitting the adjustment of desired particle size distributions.

In one embodiment of the present invention the total amounts of active pharmaceutical ingredient are provided in a dose proportional manner.

In another embodiment of the present invention the active pharmaceutical ingredient is provided in its free base form, preferably in its polymorphic form I, II or III, more preferably in its polymorphic form II or III, most preferably in its polymorphic form III.

In a further embodiment of the present invention the active pharmaceutical ingredient is provided in form of a pharmaceutically acceptable salt, preferably in form of the malate salt.

### Example

The amounts of active pharmaceutical ingredient and all excipients given in the table below refer to the contents per single tablet in one capsule. The below described granules are prepared in a dose proportional manner so that every dose strength may be obtained by compressing the same granules.

| **ingredient** | **product, e.g.** | **function** | **amount [mg]** |
|---|---|---|---|
| Sunitinib | | API | 50 |
| MCC | Avicel® PH-102 | filler | 17 |
| PVP | Kollidon® 25 | binder | 4 |
| crospovidone | Kollidon®CL | disintegrant | 8 |
| silicified MCC | Prosolv® | binder | 10.8 |
| magnesium stearate | | lubricant | 0.8 |
| colloidal silicon dioxide | Aerosil® 200 | flow enhancer | 0.4 |

Sunitinib, MCC and 50% crospovidone are wet granulated with a solution of PVP in purified water. The resulting granules are dried, sieved and mixed with magnesium stearate, colloidal silicon dioxide, silicified MCC and the residual 50% crospovidone. The mixture is compressed to a small tablet such that the weight of the granules compressed determines the final content of active ingredient in the tablet which is 12.5, 25, 37.5 or 50 mg. The resulting small tablets are individually filled into capsules.

The dissolution of these capsules is faster than that of the reference product (Sutent® capsules). After 5, 10, 15 and 30 minutes 59%, 92.9%, 96.6% and 98.1% of the active pharmaceutical ingredient are dissolved from the formulation, compared to only 28.1%, 72.3%, 86.6% and 95% from the reference product.

## Claims

1. Pharmaceutical composition comprising N-[2-(diethylamino)ethyl]-5-[(5-fluoro-1,2-dihydro-2-oxo-3H-indol-3-ylidene)methyl]-2,4-dimethyl-1H-pyrrole-3-carboxamide as active pharmaceutical ingredient, wherein the active pharmaceutical ingredient is contained in micro tablets.

2. The pharmaceutical composition according to claim 1, wherein the micro tablet(s) are contained in a capsule shell.

3. The pharmaceutical composition according to claim 1 or 2, wherein the active pharmaceutical ingredient is present in an amount of 10 to 90 %, preferably 20 to 80 . %, more preferably 30 to 70 % most preferably 45 to 65 % by weight of the total weight of the composition.

4. The pharmaceutical composition according to any of the preceding claims, wherein active pharmaceutical ingredient is present in a total amount of 12.5, 25, 37.5 or 50 mg per single dosage form.

5. The pharmaceutical composition according to any of the preceding claims, wherein the total amounts of active pharmaceutical ingredient are provided for in a dose proportional manner.

6. The pharmaceutical composition according to any of the preceding claims, wherein the active pharmaceutical ingredient is provided in its free base form, preferably in its polymorphic form I, II or III, more preferably in its polymorphic form II or III, most preferably in its polymorphic form III.

7. The pharmaceutical composition according to any of claims 1-5, wherein the active pharmaceutical ingredient is provided in form of a pharmaceutically acceptable salt, preferably in form of the malate salt.
